# EUROPÄISCHE PATENTANMELDUNG

(11) **EP 2 145 616 A1**
(43) Veröffentlichungstag der Anmeldung: **20.01.2010**
(21) Anmeldenummer: 08012988.5
(22) Anmeldetag: 18.07.2008
(51) Int. Cl.: A61K 8/87, A61Q 5/06, A61Q 5/10

(54) **PUD für Haarfärbemittel**

(71) Anmelder: Bayer MaterialScience AG, 51368 Leverkusen (DE)
(72) Erfinder: Viala, Sophie, Dr., 50935 Köln (DE); Dörr, Sebastian, Dr., 40597 Düsseldorf (DE); Hofacker, Steffen, Dr., 51519 Odenthal (DE)

(57) **Zusammenfassung**

Die vorliegende Erfindung betrifft Haarfärbemittel, enthaltend spezielle Polyurethandispersionen sowie die Verwendung der genannten Polyurethandispersionen zur Herstellung von Haarfärbmittel-Zusammensetzungen.

## Beschreibung

Die vorliegende Erfindung betrifft Haarfärbemittel, enthaltend spezielle Polyurethane sowie die Verwendung der genannten Polyurethane zur Herstellung von Haarfärbmittel-Zusammensetzungen.

Zur Färbung von Haaren werden unterschiedliche Produkte angeboten: temporäres direktziehendes, semipermanentes und permanentes Färbemittel. Diese Haarfärbmittel unterscheiden sich von ihrem Färbungsmechanismus und ihrer Verbrauchereigenschaften.

Die Verwendung von direktziehenden temporären Haarfärbemitteln erzielt nur eine bestimmte Nuance einer vorhandenen Haarfarbe. Die verwendeten Farbstoffe (zum Beispiel Lebensmittel-oder Pflanzenfarbstoffe) werden auf der Oberfläche des Haares abgelagert. Daher werden diese Haarfärbmittel mit Shampoo leicht entfernt.

Bei dem semipermanenten Färbemittel werden meistens nichtionische oder kationische Stoffe mit niedrigem Molekulargewicht eingesetzt, die das Haar durch Eindiffundieren ohne chemische Strukturveränderung färben. Im Gegensatz zu den direktziehenden temporären Haarfärbemitteln sind sie weniger leicht entfernbar. Sie überstehen 5 bis 6 Haarwäschen.

Im Fall von permanenten Haarfarbmitteln, auch Oxidationsfarbstoffe genannt, werden die Farbstoffe direkt auf und im Haar durch chemische Reaktionen erzeugt und gebunden. Die Oxidationsfarbstoffe sind farblose Vorstufen. Durch Wasserstoffperoxid als Oxidationsmittel finden Oxidationsreaktionen und Kupplungsreaktionen bzw. Kondensationen statt. Daraus resultieren die gefärbten Verbindungen unterschiedlichen Polymerisationsgrads.

Die oben genannten Haarfärbmittel haben ihre Nachteile und Vorteile. Da die Färbung von Haaren bei den Oxidationsfarbstoffen durch den Einsatz von starken Oxidationsmitteln wie zum Beispiel Wasserstoffperoxid geleistet wird, werden die Haare stark belastet. Dieses hat Schädigungen der Haare zur Folge. Andererseits sind die direktziehenden temporären Haarfärbemittel schonend für die Haare. Jedoch sind die Ergebnisse der Färbung von geschädigten Haaren ohnehin häufig nicht zufriedenstellend. Außerdem sind diese Haarfärbmittel wenig resistent gegen Waschen. Deshalb enthalten viele Produkte eine Kombination aus verschiedenen Farbstofftypen um die gewünschten Eigenschaften wie zum Beispiel natürliche Farbtöne zu erzielen.

EP 1695688 beschreibt wasserfreie Formulierungen enthaltend ein filmbildendes Polymer und mindestens ein direktziehendes kationisches oder nichtionisches Haarfärbmittel. Wässrige Polyurethandispersionen werden hingegen nicht erwähnt.

Aus WO 99/36047 sind Zusammesetzungen aus 2 Teilen Oxidationshaarfarbemittel und nichtionischen Polyetherpolyurethanen bekannt. Die Werwendung oder die Vorzüge von wässrigen Polyurethandispersionen sind nicht beschrieben.

Es wurde nun überraschenderweise gefunden, dass durch Einsatz von wässrigen Polyurethan-dispersionen ein verbessertes, langanhaltendes Färbeergebnis erzielt werden kann.

Gegenstand der vorliegenden Erfindung sind daher kosmetische Zusammensetzungen zum Färben keratinhaltiger Materialien, bevorzugt der Haare, enthaltend eine oder mehrere wässrige Polyurethan-Dispersionen sowie kosmetische Zusammensetzungen zum Färben der Haare, bei deren Herstellung eine oder mehrere wässrige Polyurethan-Dispersionen eingesetzt werden.

Polyurethane im Sinne der Erfindung sind polymere Verbindungen, die mindestens zwei UrethanGruppen-haltige Wiederholungseinheiten aufweisen:

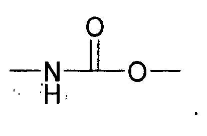

Erfindungsgemäß sind auch solche Polyurethane eingeschlossen, die herstellungsbedingt auch Harnstoff-Gruppen-haltige Wiederholungseinheiten: 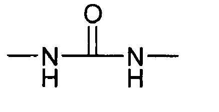 aufweisen, wie sie insbesondere bei der Kettenverlängerung isocyanatterminierter Prepolymere mit Polyaminen gebildet werden.

Die Verwendung dieser Polyurethane als wässrige Dispersionen führt überraschend zu einer Verbesserung der Färbung und einer langanhaltenden Dauer des Färbeeffektes.

In den erfindungsgemäßen kosmetischen Zusammensetzungen liegt das Polyurethan im Allgemeinen in dispergierter, d.h. nicht-gelöster Form vor. Bei den erfindungsgemäßen kosmetischen Zusammensetzungen handelt es sich insbesondere um wasser-enthaltende, d.h. wässrige Zusammensetzungen, in denen das Polyurethan dispergiert vorliegt. Wasser bildet im Allgemeinen neben wahlweise vorhandenen anderen flüssigen Medien, wie beispielsweise Lösungsmitteln, den Hauptbestandteil (> 50 Gew.-%) bezogen auf die flüssigen Dispergiermedien in den erfindungsgemäßen kosmetischen Zusammensetzungen, gegebenenfalls auch das alleinige flüssige Dispergiermedium.

Die in den erfindungsgemäßen kosmetischen Zusammensetzungen enthaltenen Polyurethane enthalten bevorzugt eine Sequenz, die ausgewählt wird aus der Gruppe, die besteht aus: Polyester-,. Polyether-, Polycarbonat- und Polyether-Polycarbonat-Sequenzen. Dies bedeutet erfindungsgemäß, dass die Polyurethane Ester-, Ether- und/oder Carbonat-haltige Wiederholungseinheiten enthalten.

Die erfindungsgemäßen Polyurethane sind bevorzugt im Wesentlichen lineare Moleküle, können jedoch auch verzweigt sein, was weniger bevorzugt ist.

Das zahlenmittlere Molekulargewicht der erfindungsgemäß bevorzugt verwendeten Polyurethane beträgt typischerweise von 1000 bis 100000, bevorzugt von 5000 bis 50000 g/mol.

Die in den erfindungsgemäßen kosmetischen Zusammensetzungen enthaltenen Polyurethane werden den genannten Zusammensetzungen insbesondere als wässrige Dispersionen zugesetzt. Derartige wässrige Polyurethan-Dispersionen sind insbesondere erhältlich durch ein Verfahren, welches die Umsetzung folgender Komponenten umfasst:
A1) ein oder mehrere organische Polyisocyanate,
A2) einem oder mehreren polymeren Polyolen, bevorzugt mit zahlenmittleren Molekulargewichten von 400 bis 8000 g/mol (hier und bei den folgenden Molekulargewichtangaben bestimmt durch Gelpermeationschromatographie gegenüber Polystyrol-Standard in Tetrahydofuran bei 23°C), bevorzugter 400 bis 6000 g/mol und besonders bevorzugt von 600 bis 3000 g/mol, und OH-Funktionalitäten von bevorzugt 1,5 bis 6, bevorzugter 1,8 bis 3, besonders bevorzugt von 1,9 bis 2,1,
A3) gegebenenfalls ein oder mehrere hydroxyfunktionelle Verbindungen, bevorzugt mit Molekulargewichten von 32 bis weniger als 400 g/mol,
A4) gegebenenfalls ein oder mehrere isocyanatreaktive Hydrophilierungsmittel, welche bevorzugt aus anionischen, potentiell anionischen und/oder nichtionischen Hydrophilierungsmitteln ausgewählt werden, und
B1) gegebenenfalls ein oder mehrere aminofunktionelle Verbindungen, bevorzugt mit Molekulargewichten von 32 bis 400 g/mol.

Die in den erfindungsgemäßen, kosmetischen Zusammensetzungen enthaltenen wässrigen Polyurethan-Dispersionen sind bevorzugter erhältlich durch ein Verfahren, welches die folgenden Verfahrensschritte umfasst:

Verfahren zur Herstellung eines isocyanatfunktionellen Prepolymers, welches die Umsetzung folgender Komponenten umfasst:
A1) ein oder mehrere organische Polyisocyanate,
A2) polymeren Polyolen, bevorzugt mit zahlenmittleren Molekulargewichten von 400 bis 8000 g/mol (hier und bei den folgenden Molekulargewichtangaben bestimmt durch Gelpermeationschromatographie gegenüber Polystyrol-Standard in Tetrahydofuran bei 23°C), bevorzugter 400 bis 6000 g/mol und besonders bevorzugt von 600 bis 3000 g/mol, und OH-Funktionalitäten von bevorzugt 1,5 bis 6, bevorzugter 1,8 bis 3, besonders bevorzugt von 1,9 bis 2,1,
A3) gegebenenfalls ein oder mehrere hydroxyfunktionelle Verbindungen, bevorzugt mit Molekulargewichten von 32 bis weniger als 400 g/mol,
A4) gegebenenfalls ein oder mehrere isocyanatreaktive Hydrophilierungsmittel, welche bevorzugt aus anionischen, potentiell anionischen und/oder nichtionischen Hydrophilierungsmitteln ausgewählt werden,
(wobei die Stöchiometrie so gewählt wird, dass die entstehenden Prepolymere isocyanatterminiert sind), und

Verfahren zur Herstellung des Polyurethans, welches umfasst:

Die Umsetzung des in Schritt A) erhaltenen isocyanatfunktionellen Prepolymers mit einer oder mehreren der Komponenten, welche aus der Gruppe ausgewählt werden, die besteht aus:
B1) ein oder mehreren aminofunktionellen Verbindungen, bevorzugt mit Molekulargewichten von 32 bis 400 g/mol und
B2) ein oder mehreren isocyanatreaktiven, bevorzugt aminofunktionellen Hydrophilierungsmitteln, welche bevorzugt aus anionischen und potentiell anionischen Hydrophilierungsmitteln ausgewählt werden.

In Schritt B) erfolgt im Allgemeinen eine Kettenverlängerung des in Schritt A) erhaltenen isocyanatfunktionellen Prepolymers.

Insbesondere umfasst die aminofunktionelle Verbindung B) wenigstens eine aminofunktionelle Verbindung B1), die keine ionischen und/oder ionogene Gruppen aufweist. Hierbei handelt es sich bevorzugt um ein Diamin, welches keine ionischen und/oder ionogenen Gruppen aufweist.

Bevorzugt werden in Schritt B) sowohl die Komponente B1) als auch die Komponente B2) verwendet.

Die Dispergierung der Polyurethane in Wasser kann vor, während oder nach Schritt B) erfolgen, wobei gegebenenfalls enthaltene, potentiell ionische Gruppen durch teilweise oder vollständige Umsetzung mit einem Neutralisationsmittel in die ionische Form überführt werden.

Um die erfindungsgemäß bevorzugte anionische Hydrophilierung zu erreichen, müssen die anionischen oder potentiell anionischen Komponenten A4) und/oder B2) als Hydrophilierungsmittel eingesetzt werden, was bevorzugt der Fall ist. Diese weisen bevorzugt wenigstens eine gegenüber NCO-Gruppen reaktive Gruppe wie bevorzugt eine Amino-, Hydroxy- oder Thiolgruppen auf und darüber hinaus wenigstens eine anionische Gruppe oder in eine anionische Gruppe überführbare Gruppe auf, wie -COO⁻, -SO₃⁻ oder -PO₃²⁻ bzw. deren ganz oder teilweise protonierte Säureformen. Dies wird weiter unten noch näher ausgeführt. Besonders bevorzugt sind Sulfonatgruppen als anionische Gruppen. Die Verwendung von Sulfonatgruppen als anionische Gruppen in den erfindungsgemäß verwendeten Polyurethanen führt zu einer größeren Beständigkeit gegenüber Elektrolyten, wie Salzen, die Einstellung eines neutralen pH-Wertes der Dispersionen wird vereinfacht, und die Dispersionenen bleiben über einen breiteren pH-Wert wie einem pH von etwa 5 bis 8 stabil. Auch wird durch die Verwendung der Sulfonatgruppen-tragenden Polyurethane die Stabilität der erfindungsgemäßen kosmetischen Zusammensetzungen erhöht.

Die bevorzugten wässrigen, anionischen Polyurethan-Dispersionen haben einen niedrigen Grad an hydrophilen anionischen Gruppen, bevorzugt von 0,1 bis 15 Milliequivalenten pro 100 g Festharz. Ein höherer Grad an anionischen Gruppen ist nachteilig weil in einigen Fällen die Trockung des resultierenden Films aus der kosmetischen Zusammensetzung verlangsamt werden könnte.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm und ganz besonders bevorzugt weniger als 400 nm, bestimmt mittels Laserkorrelations-Spektroskopie nach Verdünnung mit entionisiertem Wasser (Gerät: Malvern Zetasizer 1000, Malver Inst. Limited).

Im Schritt A) zur Herstellung der erfindungsgemäß verwendeten Polyurethane beträgt das molare Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) bei der Herstellung des NCO-funktionellen Prepolymers bevorzugt 1,05 bis 3,5, bevorzugter 1,2 bis 3,0 besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers bevorzugt 40 bis 150 %, bevorzugter zwischen 50 bis 125 %, besonders bevorzugt zwischen 60 bis 120 % beträgt.

Geeignete Polyisocyanate der Komponente A1) sind insbesondere die dem Fachmann an sich bekannten aromatischen, araliphatischen, aliphatischen oder cycloaliphatischen Polyisocyanate einer NCO-Funktionalität von insbesondere 2.

Beispiele solcher geeigneten Polyisocyanate sind 1,4-Butylendüsocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), sowie Alkyl-2,6-diisocyanatohexanoate (Lysindiisocyanate) mit C1-C8-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül sei z.B. 4-Isocyanatomethyl-1,8-octandiisocyanat (Nonantriisocyanat) oder Triphenylmethan-4,4',4''-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugter 2 bis 2,6 und besonders bevorzugt 2 bis 2,4, ganz besonders bevorzugt 2. Eine Funktionalität von ungefähr 2 ist bevorzugt, weil dadurch geeignete mechanische Eigenschaften resultieren.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

In A2) werden polymere Polyole mit einem zahlenmittleren Molekulargewicht Mₙ von bevorzugt 400 bis 8000 g/mol, bevorzugter von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Der Ausdruck "polymere" Polyole bedeutet hier insbesondere, dass die genannten Polyole mindestens zwei, bevorzugter mindestens drei miteinander verbundene Wiederholungseinheiten aufweisen.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Die bevorzugt verwendeten Polyesterpolyole sind die an sich bekannten Polykondensate aus Disowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Erfindungsgemäß besonders bevorzugt sind als Komponente A2) zur Herstellung der Polyurethane, Polyesterpolyole mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol.

Ebenfalls können als Komponente A2) hydroxylgruppenaufweisende Polycarbonate, bevorzugt Polycarbonatdiole, mit zahlenmittleren Molekulargewichten Mₙ von bevorzugt 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Hydroxylgruppen aufweisende Polycarbonate sind bevorzugt linear gebaut.

Ebenfalls können als Komponente A2) Polyetherpolyole eingesetzt werden.

Besonders geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen-und/oder Polybutylenglykole anwendbar, insbesondere mit den oben genannten bevorzugten Molekulargewichten.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Besonders bevorzugte Komponenten in A2) sind Polytetramethylenglykolpolyether und deren Mischungen mit anderen Polyolen und besonders bevorzugt sind ausschließlich Polytetramethylenglykolpolyether.

Besonders bevorzugt werden ausschließlich Polytetramethylenglykolpolyether eingesetzt, dies aber als Mischungen von mindestens zwei Polytetramethylenglykolpolyethern mit unterschiedlichen mittleren Molekulargewichten. Ganz besonders bevorzugt sind Mischungen von zwei Fraktionen Polytetramethylenglykolpolyether, wobei die Fraktion dem niedrigeren Molekulargewicht zu 3 bis 80 Gew.-%, bevorzugt zu 8 bis 30 Gew.-% und ganz besonders bevorzugt von 14 bis 25 Gew.-% bezogen auf die Gesamtmenge der eingesetzten Polytetramethylenglykolpolyether vorliegt.

Das zahlenmittlere Molekulargewicht Mₙ der Fraktion mit dem niedrigeren Molekulargewicht liegt bevorzugt im Bereich von 400 bis 2000 g/mol, besonders bevorzugt von 650 bis 1400 g/mol, ganz besonders bevorzugt um 1000 g/mol. Das zahlenmittlere Molekulargewicht Mₙ der Fraktion mit dem höheren Molekulargewicht liegt bevorzugt im Bereich von 1000 bis 8000 g/mol, besonders bevorzugt von 1500 bis 4000 g/mol, ganz besonders bevorzugt um 2000 g/mol.

In einer bevorzugten Ausführungsformen der Erfindung enthält Komponente A2) demnach:
- wenigstens ein Poly(tetramethylenglykol)polyetherpolyol (wie (HO-(CH₂-CH₂-CH₂-CH₂-O)ₓ-H),
- besonders bevorzugt keine anderen Komponente als Poly(tetramethylenglykol)-polyetherpolyole (wie (HO-(CH₂-CH₂-CH₂-CH₂O)ₓ-H),
- ganz besonders bevorzugt zwei Fraktionen Polytetramethylenglykolpolyether, wobei die Fraktion mit dem niedrigeren Molekulargewicht zu 3 bis 80 Gew.-% und bevorzugt zu 8 bis 30 Gew.-% und ganz besonders bevorzugt von 14 bis 25 Gew.-% bezogen auf die Gesamtmenge der eingesetzten Polytetramethylenglykolpolyether enthalten ist und
die Komponente A) definitionsgemäß im Wesentlichen weder ionische noch ionogene Gruppen aufweist.

Die gegebenenfalls als Komponente A3) verwendeten hydroxyfunktionellen Verbindungen besitzen zweckmäßig Molekulargewichte von 62 bis weniger als 400 g/mol. Im Unterschied zu den Komponenten A2) handelt es sich bei der Komponente A3) insbesondere nicht um polymere hydrofunktionelle Verbindungen sondern um monomere Verbindungen, welche keine Wiederholungseinheiten wie die Verbindungen der Komponente A2) aufweisen.

Als Komponente A3) können insbesondere Polyole, bevorzugt des genannten Molekulargewichtsbereichs, bevorzugt mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclo-hexyl)propan), Trimethylolpropan, Glycerin, Pentaerythrit sowie deren beliebige Mischungen untereinander eingesetzt werden.

Geeignet sind als Komponente A3) auch Esterdiole des genannten Molekulargewichtsbereichs wie α-Hydroxybutyl-ε-hydroxycapronsäureester, ω-Hydroxyhexyl-γ-hydroxybuttersäure-ester, Adipinsäure-(β-hydroxyethyl)ester oder Terephthalsäurebis(β-hydroxyethyl)-ester.

Ferner können als Komponente A3) auch monofunktionelle, isocyanatreaktive, Hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykol-monobutylether, Propylenglykolmonomethylether, Dipropylenglykol-monomethylether, Tripropylenglykolmonomethylether, Dipropylenglykolmonopropylether, Propylenglykol-monobutylether, Dipropylenglykolmonobutylether, Tripropylenglykolmonobutylether, 2-Ethylhexanol, 1-Octanol, 1-Dodecanol, 1-Hexadecanol.

Bevorzugte Verbindungen der Komponente A3) sind 1,6-Hexandiol, 1,4-Butandiol, Neopentylglykol und Trimethylolpropan.

In einer bevorzugten Ausführungsform der Erfindung enthält dass erfindungsgemäß verwendete Polyurethan weniger als etwa 10 Gew.-% der Komponente A3), bevorzugt weniger als 5 Gew.-% der Komponente A3), jeweils bezogen auf die Gesamtmasse des Polyurethans, noch bevorzugter wird Komponente A3) zur Herstellung des Polyurethan nicht verwendet.

Als Komponente A4) werden zur Herstellung der erfindungsgemäß verwendeten Polyurethane gegebenenfalls ein oder mehrere isocyanatreaktive Hydrophilierungsmittel verwendet, welche bevorzugt aus anionischen, potentiell anionischen und/oder nichtionischen Hydrophilierungsmittein ausgewählt werden. Die als Komponente A4) verwendeten isocyanatreaktiven Hydrophilierungsmittel sind insbesondere von den Komponenten A2) und A3) verschieden.

Unter anionisch bzw. potentiell anionisch hydrophilierenden Verbindungen der Komponente A4) werden insbesondere sämtliche Verbindungen verstanden, die mindestens eine isocyanatreaktive Gruppe wie eine Hydroxylgruppe oder eine Aminogruppe aufweisen sowie mindestens eine Funktionalität wie z.B. -COO⁻M⁺, -SO₃⁻M⁺, -PO(O⁻M⁺)₂ mit M⁺ beispielsweise gleich Metallkation, insbesondere Alkalimetallkation, wie Na⁺, H⁺, NH₄⁺, NHR₃⁺, wobei R jeweils ein C1-C12-Alkylrest, C5-C6-Cycloalkylrest und/oder ein C2-C4-Hydroxyalkylrest sein kann. Diese Verbindungen gehen, wie dem Fachmann bekannt ist, bei Wechselwirkung mit wässrigen Medien ein pH-Wert-abhängiges Dissoziationsgleichgewicht ein und können auf diese Weise negativ oder neutral geladen sein. Geeignete anionisch oder potentiell anionisch hydrophilierende Verbindungen schließen insbesondere ein: Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃ wie es in DE-A 2 446 440, Seite 5 - 9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel A4) sind solche, die Carboxylat- bzw Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente A4) schließen insbesondere auch Polyoxyalkylenether ein, welche über isocyanat-reaktive Gruppen, wie Hydroxy-und/oder Aminogruppen verfügen. Bevorzugt handelt es sich um einen monofunktionellen Polyoxyalkylenether, insbesondere um einen monohydroxyfunktionellen Polyoxyalkylenether, wie beispielsweise ein monohydroxyfunktioneller Polyether auf Ethylen-/Propylenoxid-Basis, wie der von der Anmelderin vertriebene Polyether LB 25.

Beispiele der nichtionisch hydrophilierenden Verbindungen als Komponente A4) sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle, insbesondere auch monofunktioneller Alkohole, zugänglich sind (z.B. in Ullmanns Encyclopädie der technischen Chemie, 4. Auflage, Band 19, Verlag Chemie, Weinheim S. 31-38).

Diese sind insbesondere entweder reine Polyethylenoxidether oder gemischte Polyalkylenoxidether, insbesondere Polyethylenoxid-/Polypropylenoxid-Polyether-Verbindungen, die blockartig oder statistisch aufgebaut sein können, wobei sie bevorzugt mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis weniger als 100 mol-% Ethylenoxid- und mehr als 0 bis 60 mol-% Propylenoxideinheiten aufweisen.

Geeignete Startermoleküle für solche nichtionischen Hydrophilierungsmittel A4) sind insbesondere gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykol-monobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol. Weniger bevorzugt sind sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Wahlweise kann in Schritt B) die Komponente B1) verwendet werden. Die Komponente B1) wird ausgewählt aus aminofunktionelle Verbindungen, bevorzugt mit Molekulargewichten von 32 bis 400 g/mol. Die Komponente B1) ist von der Komponente A4) und der Komponenten B2) verschieden. Sie unterscheidet sich von der Komponente A4) und der Komponenten B2) bevorzugt dadurch, dass sie keine anionischen und potentiell anionischen Gruppen aufweist.

Als Komponente B1) können insbesondere Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich ist die Verwendung von Hydrazin oder sowie Hydraziden wie Adipinsäuredihydrazid. Bevorzugt sind Isophorondiamin, 1,2-Ethylendiamin, 1,4-Diaminobutan, Hydrazin und Diethylentriamin.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin.

Bevorzugt kann in Schritt B) die Komponente B2) verwendet werden. Die Komponente B2) wird ausgewählt unter isocyanatreaktiven, bevorzugt aminofunktionellen Hydrophilierungsmitteln, welche bevorzugt aus ionischen und/oder ionogenen, insbesondere anionischen und potentiell anionischen Hydrophilierungsmitteln ausgewählt werden. Dabei kann es sich um die gleichen Verbindungen handeln, die wahlweise in Schritt A) als Komponente A4) verwendet werden können, mit Ausnahme der nicht-ionischen Hydrophilierungsmittel. Als Komponente A4) und Komponente B2) können daher in einem Verfahren zur Herstellung des erfindungsgemäß verwendeten Polyurethans gleiche oder verschiedene Verbindungen verwendet werden.

Hinsichtlich der anionisch bzw. potentiell anionisch hydrophilierenden Verbindungen der Komponente B2) kann daher an sich zu den für Komponente A4) gegebenen Definitionen verwiesen werden. Die Komponente B2) schließt insbesondere sämtliche Verbindungen ein, die mindestens eine isocyanatreaktive Gruppe, bevorzugt eine Amino-Gruppe aufweisen, sowie mindestens eine anionische Funktionalität wie z.B. -COO⁻M⁺, -SO₃⁻M⁺, -PO(O⁻M⁺)₂ mit M⁺ beispielsweise gleich Metallkation, wie AlkalimetallKation, wie Na⁺, H⁺, NH₄⁺, NHR₃⁺, wobei R jeweils ein C1-C12-Alkylrest, C5-C6-Cycloalkylrest und/oder ein C2-C4-Hydroxyalkylrest sein kann. Wie dem Fachmann bekannt ist, gehen diese Gruppen bei der Wechselwirkung mit wässrigen Medien ein pH-Wert-abhängiges Dissoziationsgleichgewicht ein, und können auf diese Weise negativ oder neutral geladen sein.

Besonders als Komponente B2) geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiamin-propyl- oder -butylsulfonsäure, 1,2- oder 1,3-Propylendiamin-βethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann das aus aus WO-A 01/88006 bekannte Cyclohexylaminopropansulfonsäure (CAPS) als anionisches oder potentiell anionisches Hydrophilierungsmittel verwendet werden.

Bevorzugte anionische oder potentiell anionische Hydrophilierungsmittel der Komponente B2) sind solche der vorstehend genannten Art, die über Carboxylat- bzw. Carbonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

Das erfindungsgemäß verwendete Polyurethan enthält bevorzugt ein anionisches bzw. potentiell anionisches Hydrophilierungsmittel als Komponente A4) (dann wird es bereits bei der Prepolymerbildung umgesetzt) oder als Komponente B2) (dann wird es erst bei der Umsetzung des Prepolymers eingesetzt). Bevorzugt wird bei der Umsetzung des Prepolymers in Schritt B) ein anionisches bzw. potentiell anionisches Hydrophilierungsmittel als Komponente B2) zugesetzt. Mit anderen Worten enthält das erfindungsgemäß verwendete Polyurethan in einer bevorzugten Ausführungsform eine anionische oder potentiell anionische, d.h., in ein Polyurethan-Anion dissoziierbare Gruppe. Die besonders bevorzugte anionische bzw. potentiell anionische Gruppe ist die SulfonatGruppe: -SO₃⁻M⁺. Diese wird besonders bevorzugt mittels eines Diaminosulfonats, insbesondere mittels des NH₂-CH₂CH₂-NH-CH₂CH₂-SO₃Na in das Polyurethan eingebracht.

In einer bevorzugten Ausführungsform werden zur Herstellung der speziellen Polyurethan-Dispersionen sowohl die Komponente B1) als auch die Komponente B2) verwendet. Durch die Verwendung der Komponente B1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten B1) und B2) läßt sich insbesondere eine optimale Balance zwischen Hydrophilie und Kettenlänge einstellen.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
40 bis 90 Gew.-% A2),
0,5 bis 20 Gew.-% Summe der Komponenten A3) und/oder B1)
0,1 bis 25 Gew.-% Summe der Komponenten Komponente A4) und/oder B2), wobei bezogen auf die Gesamtmengen der Komponenten A1) bis A4) und B1) bis B2) besonders bevorzugt 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und/oder B1)
0,1 bis 15 Gew.-% Summe der Komponenten Komponente A4) und/oder B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und/oder B1),
0,1 bis 13,5 Gew.-% Summe der Komponenten A4) und/oder B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der bevorzugt anionisch hydrophilierten Polyurethan-Dispersionen kann bevorzugt in einer oder mehreren Stufe(n) in homogener oder bei mehrstufiger Umsetzung, teilweise in disperser Phase durchgeführt werden. Nach vollständig oder teilweise durchgeführter Polyaddition in Schritt A) (Komponenten A1) bis A4)) erfolgt bevorzugt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von, N-Methylpyrrolidon, N-Ethylpyrrolidon in der Dispersion verbleiben. Bevorzugt werden aber außer den üblichen aliphatischen, ketofunktionellen Lösemitteln keine anderen Lösungsmittel verwendet. Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu isocyanatreaktiven Gruppen bevorzugt 1,05 bis 3,5, bevorzugter 1,2 bis 3,0, besonders bevorzugt 1,3 bis 2,5. Bevorzugt werden also Isocyanat-funktionelle Prepolymere gebildet, welche anschließend bevorzugt mit aminofunktionellen Verbindungen B1) und/oder B2) umgesetzt werden.

Die Umsetzung der Komponenten A1) bis A4) zum Prepolymer erfolgt teilweise oder vollständig, bevorzugt aber vollständig. Es werden so bevorzugt Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldüsopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen, das erhaltene Prepolymer bevorzugt mit Hilfe von aliphatischen Ketonen wie Aceton oder 2-Butanon gelöst.

Bei der Kettenverlängerung/-terminierung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene flüchtige Lösemittel wird üblicherweise anschließend destillativ entfernt. Eine Entfernung bereits während der Dispergierung ist ebenfalls möglich.

Nach Abreaktion der Isocyanatgruppen oder nach Dispergierung können auch alkoholfunktionelle Lösemittel zugesetzt werden, beispielsweise als Koaleszenzmittel. Beispiele sind Ethanol, n-Butanol, n-Propanol, Ethylenglykolmonobutylether (2-Butoxy-ethanol), Diethylenglykolmonomethylether, Propylenglykolmonomethylether, Propylenglykolmonobutylether, Dipropylenglykolmonobutylether und Tripropylenglykolmonobutylether.

Die erfindungsgemäß bevorzugt verwendeten Polyurethan-Dispersionen sind im Allgemeinen wässrige Dispersionen. Der pH-Wert der erfindungsgemäß verwendeten Polyurethan-Dispersionen beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen, welche zur Herstellung der kosmetischen Zusammensetzung der Erfindung bevorzugt verwendet wird, beträgt im allgemeinen 20 bis 70, bevorzugt 30 bis 65, besonders bevorzugt 35 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Vorteilhaft können die erfindungsgemäßen kosmetischen Zusammensetzungen zum Färben der Haare als Shampoo, Gele, Creme, Schaumaerosol, Emulsionen oder wässrigen oder wässrigalkoholischen Lösungen vorliegen.

Die erfindungsgemäßen kosmetischen Zusammensetzungen enthalten bevorzugt 0,1 bis 20 Gew.-% der erfindungswesentlichen Polyurethandispersionen, besonders bevorzugt 0,5 bis 10 Gew.-% bezogen auf die Gesamtzusammensetzung.

Die erfindungsgemäßen kosmetischen Zusammensetzungen enthalten neben den erfindungswesentlichen Polyurethandispersionen einen oder mehrere direktziehende Farbstoffe und/oder mindestens ein Oxidationsfarbstoffvorprodukt, bevorzugt ein Gemisch aus mindestens einer Entwickler- und mindestens einer Kupplersubtanz.

Unter "direktziehenden Farbstoffen" werden bereits entwickelte Pigmente und Farbstoffe verstanden. Deren Farbentwicklung liegt bereits vor und muss nicht erst z.B. durch Oxidationsvorgänge hervorgerufen werden.

Die erfindungsgemäß verwendbaren direktziehenden Farbstoffe werden unter den neutralen, sauren oder kationischen nitrierten Benzolfarbstoffen, neutralen, sauren oder kationischen direktziehenden Azofarbstoffen, neutralen, sauren oder kationischen direktziehenden Chinonfarbstoffen und insbesondere Anthrachinon-Farbstoffen, direktziehenden Azinfarbstoffen, direktziehenden Triarylmethanfarbstoffen, direktziehenden Indoaminfarbstoffen und direktziehenden natürlichen Farbstoffen ausgewählt.

Bevorzugte direktziehende Benzolfarbstoffe sind solche ausgewählt aus der Gruppe bestehend aus:
- 1,4-Diamino-2-nitrobenzol,
- 1-Amino-2-nitro-4-β-hydroxyethylaminobenzol,
- 1-Amino-2-nitro-4-bis(β-hydroxyethyl)aminobenzol,
- 1,4-Bis(β-hydroxyethylamino)-2-nitrobenzol,
- 1-β-Hydroxyethylamino-2-nitro-4-bis(β-hydroxyethylamino)benzol,
- 1-β-Hydroxyethylamino-2-nitro-4-aminobenzol,
- 1-β-Hydroxyethylamino-2-nitro-4(ethyl)(β-hydroxyethyl)aminobenzol,
- 1-Amino-3-methyl-4-β-hydroxyethylamino-6-nitrobenzol,
- 1-Amino-2-nitro-4-β-hydroxyethylamino-5-chlorbenzol,
- 1,2-Diamino-4-nitrobenzol,
- 1-Amino-2-β-hydroxyethylamino-5-nitrobenzol,
- 1,2-Bis(β-hydroxyethylamino)-4-nitrobenzol,
- 1-Amino-2-tris(hydroxymethyl)-methylamino-5-nitrobenzol,
- 1-Hydroxy-2-amino-5-nitrobenzol,
- 1-Hydroxy-2-amino-4-nitrobenzol,
- 1-Hydroxy-3-nitro-4-aminobenzol,
- 1-Hydroxy-2-amino-4,6-dinitrobenzol,
- 1-β-Hydroxyethyloxy-2-β-hydroxyethylamino-5-nitrobenzol,
- 1-Methoxy-2-β-hydroxyethylamino-5-nitrobenzol,
- 1-β-Hydroxyethyloxy-3-methylamino-4-nitrobenzol,
- 1-β,γ-Dihydroxypropyloxy-3-methylamino-4-nitrobenzol,
- 1-β-Hydroxyethylamino-4-β,γ-dihydroxypropyloxy-2-nitrobenzol,
- 1-β,γ-Dihydroxypropylamino-4-trifluormethyl-2-nitrobenzol,
- 1-β-Hydroxyethylamino-4-trifluormethyl-2-nitrobenzol,
- 1-β-Hydroxyethylamino-3-methyl-2-nitrobenzol,
- 1-β-Aminoethylamino-5-methoxy-2-nitrobenzol,
- 1-Hydroxy-2-chlor-6-ethylamino-4-nitrobenzol,
- 1-Hydroxy-2-chlor-6-amino-4-nitrobenzol,
- 1-Hydroxy-6-bis(β-hydroxyethyl)amino-3-nitrobenzol,
- 1-β-Hydroxyethylamino-2-nitrobenzol und
- 1-Hydroxy-4-β-hydroxyethylamino-3-nitrobenzol.

Beispielweise können die direktziehenden Azofarbstoffen die kationischen Azofarbstoffe sein, die in den Patentanmeldungen WO 95/15144, WO 95/01772 und EP 714 954 beschrieben sind.

Weitere vorteilhaft sind die folgenden direktziehenden Azofarbstoffe: Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black, 9, 1-(4'-Diaminodiphenylazo)-2-methyl-4-bis(β-hydroxyethyl)aminobenzol und 4-Hydroxy-3-(2-methoxyphenylazo)-1-naphthalinsulfonsaeure, wobei sich die vorgenannten Trivialnamen auf Verbindungen gemäß International Cosmetic Ingredient Dictionary and Handbook, 12th Edition, 2008 by Tara E. Gottschalck and John E. Bailey, Ph.D. (Editor) beziehen.

Bevorzugte direktziehende Chinonfarbstoffe sind solche ausgewählt aus der Gruppe bestehend aus:
- Disperse Red 15,
- Solvent Violet 13,
- Acid Violet43,
- Disperse Violet 1,
- Disperse Violet 4,
- Disperse Blue 1,
- Disperse Violet 8,
- Disperse Blue 3,
- Disperse Red 11,
- Acid Blue 62,
- Disperse Blue 7,
- Basic Blue 22,
- Disperse Violet 15,
- Basic Blue 99,
, wobei sich die vorgenannten Trivialnamen auf Verbindungen gemäß International Cosmetic Ingredient Dictionary and Handbook, 12th Edition, 2008 by Tara E. Gottschalck and John E. Bailey, Ph.D. (Editor) beziehen,
sowie die folgenden Verbindungen:
- 1-N-Methylmorpholiniumpropylamino-4-hydroxyanthrachinon,
- 1-Aminopropylamino-4-methylaminoanthrachinon,
- 1-Aminopropylaminoanthrachinon,
- 5-β-Hydroxyethyl-1,4-diaminoanthrachinon,
- 2-Aminoethylaminoanthrachinon und
- 1,4-Bis(β,γ-dihydroxypropylamino)anthrachinon.

Von den Azinfarbstoffen sind die folgenden Verbindungen zu nennen:
- Basic Blue 17,
- Basic Red 2,
gemäß International Cosmetic Ingredient Dictionary and Handbook, 12th Edition, 2008 by Tara E. Gottschalck and John E. Bailey, Ph.D. (Editor)

Bevorzugte Triarylmethanfarbstoffe sind solche ausgewählt aus der Gruppe bestehend aus:
- Basic Green 1,
- Acid Blue 9,
- Basic Violet 3,
- Basic Violet 14,
- Basic Blue 7,
- Acid Violet 49,
- Basic Blue 26,
- Acid Blue 7.
gemäß International Cosmetic Ingredient Dictionary and Handbook, 12th Edition, 2008 by Tara E. Gottschalck and John E. Bailey, Ph.D. (Editor)

Bevorzugte Indoaminfarbstoffe sind solche ausgewählt aus der Gruppe bestehend aus:
- 2-β-Hydroxyethylamino-5-[bis(β-4'-hydroxyethyl)amino]-anilino-1,4-benz ochinon,
- 2-β-Hydroxyethylamino-5-(2'-methoxy-4'-amino)-anilino-1,4-benzochinon,
- 3-N-(2'-Chlor-4'-hydroxy)-phenyl-acetylamino-6-methoxy-1,4-benzochinon-imin
- 3-N-(3'-Chlor-4'-methylamino)-phenyl-ureido-6-methyl-1,4-benzochinon-imin,
- 3-[4'-N-(Ethyl-carbamylmethyl)-amino]-phenyl-ureido-6-methyl-1,4-benzochinin-imi n.

Natürliche Direktfarbstoffe sind aus der Gruppe von Lawson, Juglon, Alizarin, Purpurin, Karminsaeure, Kermesinsaeure, Purpurogallin, Protocatechaldehyd, Indigo, Isatin, Curcumin, Spinulosin und Apigenidin ausgewählt. Extrakte oder Absude können auch verwendet werden, die natürliche Farbstoffe enthalten, wie zum Beispiel Cataplasmen oder Extrakte auf Hennabasis.

Werden direktziehende Farbstoffe zur Färbung eingesetzt, so beträgt ihr Anteil an den erfindungsgemäßen kosmetischen Zusammensetzungen typischerweise bis zu 20 Gew.-% und bevorzugt bis zu 10 Gew.-% bezogen auf das Gesamtgewicht der Zusammensetzung

Werden Oxidationsfarbstoffvorprodukte zur Färbung eingesetzt, so setzen sich diese bevorzugt aus einer Entwickler- und einer Kupplersubstanz zusammen.

Geeignete Entwicklersubstanzen sind 4-Diaminobenzol, 2,5-Diaminotoluol, Tetraaminopyrimidine, Triaminohydroxypyrimidine, 1,2,4-Triaminobenzol, 2-(2,5- Diaminophenyl)ethanol, 2-(2'Hydroxyethylamino)5-aminotoluol und 1-Amino-4-bis-(2'-hydroxyethyl)-aminobenzol bzw. deren wasserlösliche Salze; beispielhafte Kupplersubstanzen sind Resorcin, 2-Methylresorcin, 4-Chlorresorcin, 2-Amino-4-chlorphenol, 4-(N-methyl)- aminophenol, 2-Aminophenol, 3-Aminophenol, 1-Methyl-2-hydroxy-4-aminobenzol, 3-N,N- Dimethylaminophenol, 4-Amino-3-methylphenol, 5-Amino-2-methylphenol, 6-Amino-3- methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6- Diaminopyridin, 1,3-Diaminobenzol, 1-Amino-3-(2'-hydroxyethylamino)benzol, 1-Amino-3- [bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2- amino-4-(2'-hydroxyethylamino)-benzol.

Beispielhafte Kupplersubstanzen sind Resorcin, 2-Methylresorcin, 4-Chlorresorcin, 2-Amino-4-chlorphenol, 4-(N-methyl)-aminophenol, 2-Aminophenol, 3-Aminophenol, 1-Methyl-2-hydroxy-4-aminobenzol, 3-N,N-Dimethylaminophenol, 5-Amino-2-methylphenol, 6-Amino-3-methylphenol, 3-Amino-2-methylamino-6-methoxypyridin, 2-Amino-3-hydroxypyridin, 2-Dimethylamino-S-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 4,6-Dichlorresorcin, 1, 3-Diaminotoluol, 1-Hydroxynaphthalin, 4-Hydroxy-1,2-methylendioxybenzol, 1,5-Dihydroxynaphthalin, 1,7-Dihydroxynaphthalin, 2,7-Dihydroxynaphthalin, 4-Hydroxy-1,2-methylen-dioxybenzol, 2,4-Diamino-3-chlorphenol und/oder 1-Methoxy-2-amino-4-(2'-hydroxyethylamino)benzol bzw. deren wasserlösliche Salze.

Werden Oxidationsfarbstoffvorprodukte zur Färbung eingesetzt sind Entwickler- und Kupplersubstanzen bevorzugt im Molverhältnis 1 : 3 bis 5 : 1, besonders bevorzugt 1 : 1 und 3:1, enthalten.

Werden Oxidationsfarbstoffvorprodukte zur Färbung eingesetzt so beträgt die Einsatzkonzentration von Entwickler- und Kupplersubstanzen typischerweise bis zu 5 Gew. % bezogen auf das Gesamtgewicht der Zusammensetzung je nach gewünschter Färbung.

Die erfindungsgemäßen kosmetischen Zusammensetzungen können ferner bevorzugt Verdicker enthalten.

Vorteilhafte Verdicker sind:
- Vernetzte oder nichtvernetzte Acrylsäure oder Methacrylsäure Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl-oder Vinylmonomeren abgeleitet sind, wie C10-30 Alkylacrylate, C10-30-Alkylmethacrylate und Vinylacetat.
- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure.
- Nichtionische, anionische, kationische oder amphotere assoziative Polymere z.B. auf Basis von Polyethylenglycole und ihre Derivate, oder Polyurethane.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methyl-propansulfonsäure angegeben werden.

Besonders bevorzugte Verdicker sind solche natürlicher Herkunft, vernetzte Acrylsäure oder Methacrylsäure Homo- oder Copolymere und vernetzte Copolymere von 2-Acrylamido-2-methylpro-pansulfonsäure der vorstehend genannten Art.

Ganz besonderes bevorzugte Verdicker sind Xanthangummi, wie die unter den Bezeichnungen Keltrol^{®} und Kelza^{®} von der Firma CP Kelco angebotenen Produkte oder die Produkte der Firma RHODIA mit der Bezeichnung Rhodopol und Guargummi, wie die unter der Bezeichnung Jaguar^{®} HP105 von der Firma RHODIA erhältlichen Produkte.

Ebenfalls ganz besonders bevorzugt als Verdicker sind vernetzte Homopolymere von Methacrylsäure oder Acrylsäure, die von der Firma Lubrizol unter den Bezeichnungen Carbopol^{®} 940, Car-bopol^{®} 941, Carbopol^{®} 980, Carbopol^{®} 981, Carbopol^{®} ETD 2001, Carbopol^{®} EDT 2050, Carbo-pol^{®} 2984, Carbopol^{®} 5984 und Carbopol^{®} Ultrez 10, von der Firma 3V unter den Bezeichnungen Synthalen^{®} K, Synthalen^{®} L und Synthalen^{®} MS und von der Firma PROTEX unter den Bezeichnungen Modarez^{®} V 1250 PX, Modarez^{®} V2000 PX, Viscaron^{®} A1600 PE und Viscaron^{®} A700 PE im Handel erhältlich sind.

Ebenfalls ganz besonders bevorzugt als Verdicker sind vernetzte Copolymere von Acrylsäure oder Methacrylsäure und einem C₁₀₋₃₀-Alkylacrylat oder C₁₀₋₃₀-Alkylmethacrylat und Copolymere von Acrylsäure oder Methacrylasäure und Vinylpyrrolidon. Solche Copolymere sind beispielsweise von der Firma Lubrizol unter den Bezeichnungen Carbopol^{®} 1342, Carbopol^{®} 1382, Pemulen^{®} TR1 oder Pernulen^{®} TR2 und von der Firma ISP unter den Bezeichnungen Ultrathix P-100 (INCI: Acrylic Acid/VP Crosspolymer) im Handel erhältlich.

Ebenfalls ganz besonders bevorzugt als Verdicker sind vernetzte Copolymere von 2-Acrylamido-2-methylpropansulfonsäure. Solche Copolymere sind beispielsweise von der Firma Clariant unter den Bezeichnungen Aristoflex^{®} AVC (INCI: Ammonium Acryloyldimethyltaurat/VP Copolymer) erhältlich

Falls die Verdicker verwendet werden, sind sie typischerweise in einer Konzentration von bis zu 2 Gew.-%, bevorzugt bis zu 1 Gew.-% bezogen auf das Gesamtgewicht der Zusammensetzung vorhanden.

Es ist gegebenenfalls vorteilhaft, die anionischen Polymerverdicker, sofern als Verdicker eingesetzt, zur Verbesserung ihrer Wasserlöslichkeit bzw. ihrer Wasserdispergierbarkeit mit geeigneten Basen zu neutralisieren.

Als Neutralisationsmittel für Polymere die Säuregruppen enthalten können folgende Basen eingesetzt werden: Hydroxide, deren Kation ein Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS).

Ferner enthalten die erfindungsgemäßen kosmetischen Zusammensetzungen Wasser und gegebenenfalls ein kosmetisch geeignetes Lösungsmittel. Bevorzugte kosmetisch geeignete Lösungsmittel sind aliphatische Alkohole mit C2-4 Kohlenstoffatomen wie Ethanol, Isopropanol, t-Butanol, n-Butanol; Polyol wie Propylenglycol, Glycerin, Ethylenglycol und Polyolethern; Aceton; unverzweigte oder verzweigte Kohlenwasserstoffe wie Pentan, Hexan, Isopentan und zyklische Kohlenwasserstoffe wie Cyclopentan und Cyclohexan; und deren Gemischen ausgewählt.

In den erfindungsgemäßen kosmetischen Zusammensetzungen können des weiteren haarpflegende Wirkstoffe verwendet werden. Als Pflegestoffe können bevorzugt cyclische Polydimethylsiloxane (Cyclomethicone) in Konzentrationen von 0 bis 1,0 Gew.-% der Gesamtformulierung oder Silikontenside (Polyethermodifizierte Siloxane) vom Typ Dimethicone Copolyol oder Simethicon in Konzentrationen 0 bis 1,0 Gew.-% des Gesamtgewichts der Zusammensetzung zum Einsatz kommen.

Cyclomethicone werden u. a. unter der Handelsbezeichnungen Abil^{®} K4 von der Firma Goldschmidt oder z.B. DC 244, DC 245 oder DC 345 von der Firma Dow Corning angeboten. Dimethicon-Copolyole werden z. B. unter der Handelsbezeichnung DC 193 von der Firma Dow Corning bzw. Belsil^{®} DM 6031 von der Firma Wacker angeboten.

Ferner können herkömmliche Additive in den erfindungsgemäßen kosmetischen Zusammensetzungen enthalten sein, um beispielsweise der Zusammensetzung bestimmte Modifizierungseigenschaften zu verleihen.

Dies können sein Silikone oder Silikonderivate, Benetzungsmittel, Feuchthaltemittel, Weichmacher wie Glycerin, Glykol und Phthalester und -ether, Riechstoffe und Parfüms, UV-Absorber, Farbstoffe, Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Tenside, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner.

Sofern vorhanden sind solche Additive typischerweise in Mengen von 0,001 Gew.-% bis 15 Gew.-% bevorzugt 0,01 Gew.-% bis 10 Gew.-% bezogen auf das Gesamtgewicht der kosmetischen Zusammensetzung vorhanden.

Der pH-Wert der erfindungsgemäßen kosmetischen Zusammensetzungen kann sowohl im alkalischen d.h. von 7,1 bis 11, im neutralen oder schwach sauren d.h. von 5 bis 6,9 Bereich liegen

Die erfindungsgemäßen kosmetischen Zusammensetzungen können vorteilhafter Weise in Form einer Lotion, einer Creme oder eines Schaums vorliegen und durch manuelles Aufstreichen appliziert werden. Aber auch eine Sprühapplikation mittels Pumpspray oder Aerosol ist möglich.

Die erfindungsgemäßen kosmetischen Zusammensetzungen können vorteilhaft mit einem Treibgas aufgeschäumt werden.

Dementsprechend sind die erfindungsgemäßen kosmetischen Zusammensetzungen in Form einer Lotion, einer Creme oder eines Schaumes ebenfalls ein Gegenstand der Erfindung. Darüber hinaus ist ein Gegenstand der Erfindung Pumpspray-, Aerosolverpackungs- und Schaumspender auf Pumpspray- oder Aerosolverpackungsbasis, welche die erfindungsgemäße Haarfärbemittel-Zusammensetzung enthalten, ebenfalls ein Gegenstand der Erfindung.

### Beispiele:

Sofern nicht abweichend gekennzeichnet, beziehen sich alle Prozentangaben auf das Gewicht.

Sofern nicht abweichend vermerkt, beziehen sich alle analytischen Messungen auf Messungen bei Temperaturen von 23°C.

Die Feststoff- bzw. Festkörpergehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

### Verwendete Substanzen und Abkürzungen:

- Diaminosulfonat:: NH₂-CH₂CH₂-NH-CH₂CH₂-SO₃Na (45 %ig in Wasser)
- Desmophen^{®} 2020/C2200:: Polycarbonatpolyol, OH-Zahl 56 mg KOH/g, zahlenmittleres Mo- lekulargewicht 2000 g/mol (Bayer MaterialScience AG, Leverku- sen, DE)
- PolyTHF^{®} 2000:: Polytetramethylenglykolpolyol, OH-Zahl 56 mg KOH/g, zahlen- mittleres Molekulargewicht 2000 g/mol (BASF AG, Ludwigsha- fen, DE)
- PolyTHF^{®} 1000:: Polytetramethylenglykolpolyol, OH-Zahl 112 mg KOH/g, zahlen- mittleres zahlenmittleres Molekulargewicht 1000 g/mol (BASF AG, Ludwigshäfen, DE)
- Polyether LB 25:: monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE)

### Beispiel 1: Polyurethan-Dispersion 1

987,0 g PolyTHF^{®} 2000 (Komponente A2)), 375,4 g PolyTHF^{®} 1000 (Komponente A2)), 761,3 g Desmophen^{®} C2200 (Komponente A2)) und 44,3 g Polyether LB 25 (Komponente A4)) wurden in einer Standard-Rührapparatur auf 70°C aufgeheizt. Anschließend wurde ein Gemisch aus 237,0 g Hexamethylendiisocyanat (Komponente A1)) und 313,2 g Isophorondiisocyanat (Komponente A1)) zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 25,1 g Ethylendiamin (Komponente B1)), 116,5 g Isophorondiamin (Komponente B1)), 61,7 g Diaminosulfonat (Komponente B2)) und 1030 g Wasser zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgende Eigenschaften:

| | |
|---|---|
| Feststoffgehalt: | 61% |
| Partikelgröße (LKS): | 312 nm |
| Viskosität (Viskosimeter, 23°C): | 241 mPas |
| pH (23°C): | 6,02 |
| pH (23°C): | 7,15 |

### Beispiel 2: Polyurethan-Dispersion 2

450 g PolyTHF^{®} 1000 (Komponente A2)) und 2100 g PolyTHF^{®} 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin (Komponente B1)), 143,2 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 56 % |
| Partikelgröße (LKS): | 276 nm |
| Viskosität: | 1000 mPas |

### Beispiel 3: Polyurethan-Dispersion 3

1649,0 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 291,7 g Hexamethylendiisocyanat (Komponente A1)) zugegeben und solange bei 100-115°C gerührt bis der theoretische NCO-Wert unterschritten war. Das fertige Prepolymer wurde mit 3450 g Aceton bei 50°C gelöst und anschließend eine Lösung aus 16,8 g Ethylendiamin (Komponente B1)), 109,7 g Diaminosulfonat (Komponente B2)) und 425 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42 % |
| Partikelgröße (LKS): | 168 nm |
| Viskosität: | 425 mPas |
| pH-Wert: | 7,07 |

### Beispiel 4: Polyurethan-Dispersion 4

340 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 60,1 g Hexamethylendiisocyanat (Komponente A1)) zugegeben und solange bei 105°C gerührt bis der theoretische NCO-Wert unterschritten war. Das fertige Prepolymer wurde mit 711 g Aceton bei 50°C gelöst und anschließend eine Lösung aus 2,1 g Ethylendiamin (Komponente B1)), 32,4 g Diaminosulfonat (Komponente B2)) und 104,3 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 40 % |
| Partikelgröße (LKS): | 198 nm |
| Viskosität: | 700 mPas |
| pH-Wert: | 6,31 |

### Beispiel 5: Polyurethan-Dispersion 5

450 g PolyTHF^{®} 1000 (Komponente A2)) und 2100 g PolyTHF^{®} 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 351 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42 % |
| Viskosität: | 1370 mPas |

### Anwendungstechnische Beispiele:

### Temporäres direktziehendes Färbemittel

| | 1 Gew.-% | 2 Gew.-% | 3 Gew.-% |
|---|---|---|---|
| Erfindungsgemäßes Polyurethan | 10 | 2,0 | 1,0 |
| Ethanol | | 30,0 | |
| Basic Blue 99 (CI 56059) | 0,10 | 0,035 | 0,10 |
| Basic Brown 16 (CI 12250) | 0,20 | 0,005 | 0,20 |
| Basic Brown 17 (CI 12251) | 0,40 | 0,5 | 0,30 |
| Basic Yellow 57 (CI 12719) | 0,15 | | 0,35 |
| Xanthan gum | | | 2,00 |
| Konservierungsmittel | q.s. | q.s. | q.s. |
| Parfüm | q.s. | q.s. | q.s. |
| Wasser | Ad.100 | ad. 100 | Ad.100 |

### Permanentes Färbemittel

| | 1 | 1 |
|---|---|---|
| | Gew.% | Gew.% |
| Erfindungsgemäßes Polyurethan | 2,0 | 5,0 |
| Cetyl alcohol | 5,0 | 5,0 |
| Stearyl alcohol | 2,0 | 2,0 |
| Lanolin | 1,5 | 1,5 |
| PEG-20 stearate | 1,5 | 1,5 |
| Oleth-5 | 1,0 | 1,0 |
| Ammonium sulfate | 0,50 | 0,50 |
| Sodium sulfite | 0,5 | 0,5 |
| Ammonia, 25% | 7,0 | 7,0 |
| Konservierungsmittel | q.s. | q.s. |
| Parfüm | q.s. | q.s. |
| Wasser | Ad.100 | Ad.100 |
| Oxidationsfarbstoffmischung : | | |
| 2,5,6-Triamino-4-hydroxypyrimidinsulfat | 0,01 | 0,01 |
| 2,5-Diaminotoluolsulfat | 0,55 | 0,55 |
| 4-Chlororresorcin | 0,17 | 0,17 |
| 3-Aminophenol | 0,03 | 0,03 |

## Patentansprüche

1. Kosmetische Zusammensetzungen zum Färben keratinhaltiger Materialien, enthaltend eine oder mehrere wässrige Polyurethan-Dispersionen.

2. Kosmetische Zusammensetzungen zum Färben keratinhaltiger Materialien, bei deren Herstellung eine oder mehrere wässrige Polyurethan-Dispersionen eingesetzt werden.

3. Kosmetische Zusammensetzungen gemäß Anspruch 1 oder 2, **dadurch gekennzeichnet, dass** es sich bei den keratinhaltigen Materialien um menschliches oder tierisches Haar handelt.

4. Kosmetische Zusammensetzungen gemäß einem der Ansprüche 1 bis 3, **dadurch gekennzeichnet, dass** die wässrigen Polyurethandispersionen aus
A1)ein oder mehreren organischen Polyisocyanaten,
A2)einem oder mehreren polymeren Polyolen mit zahlenmittleren Molekulargewichten von 400 bis 8000 g/mol bestimmt durch Gelpermeationschromatographie gegenüber Polystyrol-Standard in Tetrahydofuran bei 23°C und OH-Funktionalitäten von 1,5 bis 6,
A3)gegebenenfalls ein oder mehreren hydroxyfunktionelle Verbindungen mit Molekulargewichten von 32 bis weniger als 400 g/mol,
A4)gegebenenfalls ein oder mehreren isocyanatreaktiven Hydrophilierungsmitteln, welche bevorzugt aus anionischen, potentiell anionischen und/oder nichtionischen Hydrophilierungsmitteln ausgewählt werden, und
B1)gegebenenfalls ein oder mehreren aminofunktionellen Verbindungen, bevorzugt mit Molekulargewichten von 32 bis 400 g/mol.

5. Kosmetische Zusammensetzungen gemäß Anspruch 4, **dadurch gekennzeichnet, dass** in A1) Polyisocyanate oder Polyisocyanatgemische mit ausschließlich aliphatisch und/oder cycloaliphatisch gebundenen Isocyanatgruppen und einer mittleren NCO-Funktionalität der Mischung von 2 bis 4 eingesetzt werden.

6. Kosmetische Zusammensetzungen gemäß Anspruch 4 oder 5, **dadurch gekennzeichnet, dass** in A2) ausschließlich Polytetramethylenglykolpolyether eingesetzt werden, wobei es sich dabei um eine Mischung aus mindestens zwei Polytetramethylenglykolpolyethern mit unterschiedlichen zahlenmittleren Molekulargewichten handelt.

7. Kosmetische Zusammensetzungen gemäß Anspruch 6, **dadurch gekennzeichnet, dass** in A2) eine Mischung aus 2 Polytetramethylenglykolpolyethern mit unterschiedlichem zahlenmittleren Molekulargewichten eingesetzt wird, wobei der Polytetramethylenglykolpolyether mit dem niedrigeren Molekulargewicht 3 bis 80 Gew.-% bezogen auf die Gesamtmenge der eingesetzten Polytetramethylenglykolpolyether ausmacht.

8. Kosmetische Zusammensetzung gemäß Anspruch 7, **dadurch gekennzeichnet, dass** das zahlenmittlere Molekulargewicht Mₙ des Polytetramethylenglykolpolyethers mit dem niedrigeren Molekulargewicht im Bereich von 650 bis 1400 g/mol und das zahlenmittlere Molekulargewicht Mₙ des Polytetramethylenglykolpolyethers mit dem höheren Molekulargewicht im Bereich von 1500 bis 4000 g/mol liegt.

9. Kosmetische Zusammensetzungen gemäß einem der Ansprüche 1 bis 8, **dadurch gekennzeichnet, dass** der Anteil an wässriger Polyurethan-Dispersion 0,1 bis 20 Gew.-% bezogen auf die Gesamtzusammensetzung ausmacht.

10. Kosmetische Zusammensetzungen gemäß einem der Ansprüche 1 bis 9, **dadurch gekennzeichnet, dass** diese einen oder mehrere direktziehende Farbstoffe und/oder mindestens ein Oxidationsfarbstoffvorprodukt enthalten.

11. Kosmetische Zusammensetzungen gemäß Anspruch 10, **dadurch gekennzeichnet, dass** im Fall von direktziehenden Farbstoffen zur Färbung deren Anteil bis zu 20 Gew.-% und im Fall der Oxidationsfarbstoffvorprodukte deren Anteil bis zu 5 Gew. % an der Gesamtzusammensetzung ausmacht.

12. Kosmetische Zusammensetzungen gemäß einem der Ansprüche 1 bis 11 in Form einer Lotion, einer Creme oder eines Schaums.

13. Pumpsprayverpackung, Aerosolverpackung oder Schaumspender, enthaltend kosmetische Zusammensetzungen gemäß einem der Ansprüche 1 bis 12.

14. Verfahrung zur Färbung von keratinhaltigen Materialien, bei dem kosmetische Zusammensetzungen gemäß einem der Ansprüche 1 bis 12 verwendet werden.

15. Verfahrung gemäß Anspruch 14, **dadurch gekennzeichnet, dass** es sich bei den keratinhaltigen Substraten um menschliche oder tierische Haare handelt.
